# EUROPÄISCHE PATENTSCHRIFT

(11) **EP 0 776 975 B1**
(45) Veröffentlichungstag und Bekanntmachung des Hinweises auf die Patenterteilung: **29.09.2004**
(21) Anmeldenummer: 96118309.2
(22) Anmeldetag: 15.11.1996
(51) Int. Cl.: C12N 15/81, C07K 14/815, C07K 14/62, C07K 14/575, C07K 19/00

(54) **Verfahren zur Nutzung des Hefe-ADH-II-Promotorsystems zur biotechnologischen Produktion heterologer Proteine in hohen Ausbeuten**
Method for use of the yeast-ADH-II promotersystem for the biotechnological production of heterologous proteins in high yield
Méthode pour l'utilisation du système promoteur de l'ADH-II de levure pour la production biotechnologique de protéins hétérologues

(30) Priorität: 28.11.1995 DE 19544233
(43) Veröffentlichungstag der Anmeldung: 04.06.1997
(73) Patentinhaber: SCHERING AKTIENGESELLSCHAFT, 13342 Berlin (DE)
(72) Erfinder: Badziong, Werner, Dr., 65812 Bad Soden (DE); Habermann, Paul, Dr., 65817 Eppstein (DE); Möller, Jörg, Dr., 65812 Bad Soden (DE); Aretz, Werner, Dr., 61462 Königstein (DE)

(56) Entgegenhaltungen:
- WO-A-96/07424
- WO-A-96/07744
- TETTRUP H V ET AL: "A PROCESS FOR THE PRODUCTION OF HUMAN PROINSULIN IN SACCHAROMYCES CEREVISIAE" BIOTECHNOLOGY AND BIOENGINEERING INCLUDING: SYMPOSIUM BIOTECHNOLOGY IN ENERGY PRODUCTION AND CONSERVATION, Bd. 35, Nr. 4, 20. Februar 1990 (1990-02-20), Seiten 339-348, XP000095652 ISSN: 0006-3592
- HARDJITO L ET AL: "RECOMBINANT PROTEIN PRODUCTION VIA FED-BATCH CULTURE OF THE YEAST SACCHAROMYCES CEREVISIAE" ENZYME AND MICROBIAL TECHNOLOGY, Bd. 15, Nr. 2, 1. Februar 1993 (1993-02-01), Seiten 120-126, XP002052463 ISSN: 0141-0229

## Beschreibung

Die Erfindung betrifft ein neues Verfahren zur Nutzung des Hefe-pα-ADH II-Promotorsystems zur biotechnologischen Produktion von Hirudin, Hirudin-Derivaten oder Fusionsproteinen enthaltend Hirudin oder ein Hirudin-Derivat.

Bei dem ursprünglich aus dem Blutegel Hirudo medicinalis isolierten Polypeptid Hirudin handelt es sich um einen hochspezifischen Thrombininhibitor mit breitem therapeutischen Potential (F. Markwardt, Biomed. Biochim. Acta 44 (1985) 1007-1013). Die benötigten Mengen können jedoch nur auf gentechnologischem Wege über transformierte Mikroorganismen hergestellt werden. Dabei hat es sich gezeigt, daß die Hefe Saccharomyces cerevisiae als Wirtsorganismus geeignet ist, um korrekt gefaltetes und voll aktives Hirudin zu produzieren (EP A1 168 342, EP A1 200 655).

Unter Hirudin sind peptidartige Thrombininhibitoren mit einer spezifischen Aktivität von mindestens 10000 AT-U/mg (Anti-Thrombin Units/mg) zu verstehen, die von den bekannten Isohirudinen der Spezies Hirudo medicinalis abgeleitet sind und wesentliche Strukturmerkmale dieser, insbesondere die charakterisistische Verknüpfung der drei Disulfidbrücken (J. Dodt et al., Biol. Chem. Hoppe-Seyler 366 (1985) 379-385), aufweisen (vgl. z.B. EP A1 158 564, EP A1 168 342, DE 34 45 517, EP A2 193 175, EP A1 200 655, EP A1 158 986, EP A1 209 061, DE 33 42 199, EP A1 171 024).

Insbesondere wird darunter ein Hirudin verstanden, wie es in der EP A1 171 024, der EP A1 158 986 und der EP A1 209 061 beschrieben ist.

Das Gen für das Alkoholdehydrogenase-Isoenzym II (ADH II) ist in Hefezellen streng reguliert. Das ADH II-Genprodukt wird nicht entdeckt, wenn dem Fermentationsmedium fermentierbare Kohlenstoffquellen wie z.B. Glucose zugesetzt sind.

Induktionsbedingungen für den ADH II-Promotor können auch in einfachen aeroben batch-Prozessen in Schüttelkolben oder in Fermentern erzielt werden, die z.B. mit einer Glucose-Konzentration von 4 % gestartet werden. Zunächst wird bei Wachstum auf Glucose bedingt durch den sog. Crabtree-Effekt mit Hilfe des Enzyms ADH I Ethanol gebildet, das wiederum nach vollständigem Verbrauch der Glucose als weitere C-Quelle dient. Nach Abbau der Glucose wird das Ethanol-abbauende Enzym ADH II induziert und die Expression von Hirudin beginnt.

Solche batch-Fermentationen bringen in der Regel nicht die für industrielle Anwendungen angestrebte hohe Raum/Zeit-Ausbeute. Erwünscht ist die Trennung der Fermentation in eine Wachstums- und eine Produktionsphase, wie sie in z.B klassischen Antibiotika-Fermentationen durch eine limitierte fed-batch-Fahrweise erzielt wird, bei der nach Etablierung einer gewissen Zelldichte eine C-Quelle wachstumslimitierend nachgefüttert wird, um unter optimalen physiologischen Bedingungen mit hoher Ausbeute das Produkt (z.B. den Sekundärmetaboliten Penicillin) zu bilden (Hersbach et al.: The Penicillins: Properties, Biosynthesis and Fermentation, in: Biotechnology of Industrial Antibiotics, S. 45-140, Hrsg. E.J. Vandamme, Marcel Dekker, New York, 1984).

Aufgrund der Regulation des ADH II-Promotorsystems wird dieses zur biotechnologischen Herstellung von rekombinanten Proteinen in der Bäckerhefe Saccharomyces cerevisiae genutzt (Price et al.: Methods in Enzymology, Vol. 185, 308-318, 1990). Die Fermentation basiert dabei, bedingt durch das Wachstum der Hefezellen, auf der natürlichen Elimination von Glucose als C-Quelle. Bei Abwesenheit von Glucose wird die Produktbildung angeschaltet und läuft bis zum Fermentationsende weiter. Dennoch scheint die Ausbeute an Produkt hinter den Erwartungen zurückzubleiben.

Tøttrup et al. (Biotechnol. Bioeng., 35, 339-348, 1990) beschreiben eine veränderte Fermentationsführung zur intrazellulären Produktion eines Humaninsulin-Fusionsproteins, die eine Glucosezufütterung mit sich anschließender Ethanolzufütterung zur optimalen Induktion des Systems beinhaltet. Ihnen gelang gegenüber einem batch-Prozeß eine beinahe Verdoppelung der volumenbezogenen Produktausbeute durch kontinuierliche Glucosezufütterung mit konstanter Rate über den gesamten Fermentationszeitraum. Eine weitere Verdoppelung schließlich wurde dadurch erreicht, daß von einem bestimmten Zeitpunkt an die Glucose-Zufütterung durch eine ebensolche von Ethanol ersetzt wurde. Aus diesem Ergebnis wurde geschlossen, daß selbst bei Glucosemangel in der Kulturbrühe, bzw. sehr niedrigen Glucose-Konzentrationen von < 20 mg/l, der hybride Promotor noch durch Glucose oder einen Metaboliten der Glykolyse teilweise reprimiert ist. Erst durch Ethanolzugabe wird dann eine vollständige Induktion erreicht.

Nutzt man das in dem Artikel von Price et al. beschriebene Expressionssystem zur Herstellung des in der europäischen Patentschrift 0 324 712 B1 genannten Genproduktes Hirudin (siehe Beispiel 1), so haben wir überraschend festgestellt, daß die spezifische, auf die Biomasse bezogene Produktausbeute durch Ethanol-Zugabe, kontinuierlich oder diskontinuierlich, abnimmt, da ein verstärktes Zellwachstum bei gleichhohem Produkttiter zu beobachten ist.

Die direkte Zugabe von Ethanol ist im Labormaßstab untersucht worden. Die zusätzliche Aufstockung eines batch-Ansatzes mit Ethanol während des Wachstums auf Ethanol hat zwar die Biomasse erhöht, jedoch im Vergleich zum Ansatz ohne Aufstockung nicht zu einer erhöhten Produkt-Konzentration geführt.

Laboransätze, die ausschließlich mit Ethanol als C-Quelle durchgeführt wurden, erbrachten im Rahmen der Meßgenauigkeit ebenfalls keine höheren spezifischen Ausbeuten (bezogen auf die optische Dichte bzw. Biomasse) als ein Vergleichsansatz mit Glucose (siehe auch Bsp. 2). Daraus läßt sich schließen, daß Ethanol alleine kein Induktor der ADH II ist.

Ebenso der Versuch, durch geregelte modellgestützte Glucose-Fütterung ein dereprimiertes Wachstum zu erzeugen (Nachfahren, d.h. Erhöhen, der Zufütterungsrate entsprechend der zunehmenden Zelldichte) und eine Ethanol-Bildung infolge des Crabtree-Effektes zu vermeiden, um dann im 2. Schritt durch eine Puls-Zugabe von Glucose mit sich anschließender starker Ethanol-Bildung infolge des Crabtree-Effektes die Expression stärker zu induzieren, brachte keine erhöhte Produktausbeute.

Überraschenderweise wurde nun eine einfache, selbstregulierende und vor allem großtechnisch durchführbare Fermentationsweise zur Nutzung des Hefe ADH II-Promotorsystems zur biotechnologischen Produktion heterologer Proteine in hohen Ausbeuten gefunden. Dieses Verfahren umfaßt die kontinuierliche Dosierung von Glucose als Kohlenstoffquelle zu einem Fermentationsansatz (Hauptkultur), in dem anfänglich keine Glucose vorhanden war. Im Laufe der Kultur werden verschiedene Phasen so durchlaufen, daß es zu einer optimalen Induktion bzw. Produktbildung kommt. Eine solche Induktion der heterologen Genexpression in Hefe unter Verwendung des ADH II-Promotorsystems ist im Lichte des Standes der Technik, z.B. der schon zitierten Literaturstelle Price et al. sehr erstaunlich, da gemäß Price et al. erst nach einer starken Glucosedepletierung der ADH II-Promotor dereprimiert wird.

Optimale Produktausbeute mit hoher spezifischer Produktivität konnten wir in unserem System erzielen, wenn direkt nach Beimpfen der Hauptkultur eine geringe Glucosemenge mit konstanter Rate kontinuierlich zugefüttert wird. Die durch die Zufütterung bedingte Volumenzunahme der Kultur kann dabei zu einer Absenkung der tatsächlichen Glucosezufütterungsrate von bis zu 25% am Ende der Kulturzeit führen. Zunächst ist Glucose für die niedrige Zellpopulation im Überschuß vorhanden, d.h. bei reprimiertem Wachstum wird infolge des Crabtree-Effektes Ethanol gebildet. Von einem bestimmten Zeitpunkt an ist die Zelldichte so hoch, daß Glucose als alleinige Kohlenstoff-Quelle nicht mehr ausreicht und ein Mischwachstum auf Glucose und Ethanol auftritt. Erst wenn dann ebenfalls das primär gebildete Ethanol verbraucht ist und weiteres (dereprimiertes) Wachstum durch Glucose limitiert wird, wird der ADH II-Promotor angeschaltet und die Produktbildung beginnt. Gegenüber dem batch-Prozeß kann so die Produktausbeute etwa verdreifacht werden.

Gegenstand der Erfindung ist somit ein Verfahren zur biotechnologischen Fermentation von heterologen Proteinen in Hefe bei dem man eine Hauptkultur mit einer Vorkultur eines Hefestammes, der das heterologe Protein exprimieren kann, animpft, unmittelbar nach dem Animpfen eine geringe Menge Glucose, z.B. 0,7-1,4, vorzugsweise 0,9-1,3 und ganz besonders bevorzugt 1,1 g Glucose/l Kulturvolumen/h mit konstanter Rate kontinuierlich zuführt, während der gesamten Fermentation aerobe Kulturbedingungen sicherstellt, indem man den Sauerstoffpartialdruck nicht unter 20% der Sättigung absinken läßt, die Fermentation nach 2 Tagen beendet und schließlich das heterologe Protein aus dem Fermentationsansatz isoliert.

Ein weiterer Gegenstand der Erfindung ist ein Verfahren gemäß dem im vorigen Absatz beschriebenem, bei dem die Medien der Vor- und Hauptkultur bis auf einen 1 %igen Glucosezusatz im Vorkulturmedium, der vorzugsweise beim Animpfen der Hauptkultur praktisch völlig verbraucht ist, äquivalent sind und einen oder mehrere komplexe Bestandteile, wie z.B. Cornsteep, Sojamehl oder Hefeextrakt enthalten. "Praktisch völlig verbraucht" bedeutet hier eine Glucose-Konzentration < 100 mg/l.

Die in den genannten Verfahren verwendeten Hefestämme können Saccharomyces cerevisiae, vorzugsweise Y79 (siehe EP 0 324 712) oder auch Kluyveromyces lactis-Stämme sein.

Das gemäß dem Verfahren herstellbare heterologe Protein kann ein Hirudin, ein Hirudin-Derivat, oder ein Fusionsprotein enthaltend Hirudin bzw. ein Hirudin-Derivat sein. Derivate bedeutet dabei unter anderem funktionelle, d.h. biologisch aktive Fragmente der Ausgangsproteine oder Mutanten mit neuen, insbesondere vorteilhaften biologischen Eigenschaften.

### Beispiel 1: Herstellung des Expressionsvektors zur rekombinanten Produktion von Hirudin in Hefe

Der Vektor pα ADH 2 (siehe Figur 2A des Artikels von Price et al.) enthält benachbart zur Restriktionsenzymschnittstelle Spe 1 in Richtung 3 Ende der c-DNA eine für das Plasmid einzigartige Erkennungsstelle für das Enzym Nco1.

Setzt man nun DNA des beschriebenen Vektors mit Kpn1 und Nco1 um, so erhält man zwei DNA-Fragmente, die gelelektrophoretisch voneinander getrennt werden. Das größere der beiden Fragmente wird isoliert und mit einem Kpn1/Nco1 Hirudinfragment, welches man aus dem Plasmid pαf Hir17 (EP 0 324 712 B1) durch partiellen Verdau mit dem Enzym Kpn 1 und vollständigem Verdau mit dem Enzym Nco 1 gewinnt, in einer T4-Ligase Reaktion umgesetzt.

Kommerziell erhältliche kompetente E.coli K12-Zellen des Stammes HB101 werden mit dem Ligationsgemisch transformiert und der Transformationsansatz auf Na-Agar-Platten, die 25 mg/l Ampicillin enthalten, ausplattiert. Die Platten werden über Nacht bei 37°C inkubiert und am nächsten Morgen werden Einzelkolonien von den Platten gepickt und zur Anzucht von Übernacht-Kulturen verwendet. Aus den Zellen der Kulturen wird jeweils Plasmid-DNA isoliert und mittels Restriktionsanalyse überprüft. Richtige Plasmid-DNA, die das Hirudingen wie gewünscht eingebaut enthält, wird zur Transformation des Saccharomyces cerevisiae-Stammes Y79, wie in EP 0 324 712 B1 beschrieben, verwendet.

Man erhält so das Expressionssystem, das für die beschriebene Verfahrensentwicklung benutzt worden ist.

### Beispiel 2: Vergleich von Laboransätzen zur rekombinanten Hirudin-Herstellung in Hefe bei Verwendung verschiedener Kohlenstoffquellen

In einem Laborfermenter werden unter sonst gleichen Bedingungen verschiedene Kohlenstoff-Quellen (C-Quellen) untersucht. Vor- und Hauptstufenmedium setzt sich wie folgt zusammen:
1 % Hefeextrakt
2 % Pepton
   C-Quelle (jeweils gleiche Menge bezogen auf C-Gehalt)
80 mg/l Adenin
80 mg/l Uracil
pH = 5,0

Die Hauptkultur wird mit 2 % Inoculum beimpft und 48 Stunden inkubiert.

Bezüglich Ethanol und Glucose als C-Quelle wurden folgende Ergebisse erzielt:

| C-Quelle | Hirudin-Konzentration [%] | optische Dichte A₅₇₈ₙₘ [%] |
|---|---|---|
| 2 % Glucose | 100 | 100 |
| 1,54 % Ethanol | 83 | 71 |

### Beispiel 3: Vergleich der rekombinanten Hirudin-Herstellung in Hefe durch Fermentation mit oder ohne Glucose-Zufütterung

### a) Fermentation mit Glucose-Zufütterung (fed-batch-Prozeß)

In einem Fermenter mit 3.600 l Gesamtvolumen werden 1600 l Kulturmedium, bestehend aus einem oder mehreren komplexen Bestandteilen, wie z.B. Cornsteep, Sojamehl, Hefeextrakt etc., ohne Zusatz von Glucose oder anderen C-Quellen, wie Saccharose, 20 Minuten bei 121°C sterilisiert.

Nach Abkühlen wird das Medium mit ca. 200 l einer ca. 12 Stunden gewachsenen Vorkultur (optische Dichte A₅₄₀ₙₘ = 3,5 ± 0,5) die nur noch sehr geringe Restmengen an Glucose enthält, beimpft. Das Medium der Vorkultur ist dem der Hauptkultur äquivalent mit der Ausnahme, daß 1 % Glucose zugesetzt wird.

Unmittelbar nach Beimpfen der Hauptstufe werden 2 kg Glucose pro Stunde in Form einer 20 % igen wässrigen Lösung mit gleichmäßiger Rate bis zum Ende der Fermentation über eine Vorrichtung zur kontinuierlichen Sterilisation zudosiert.

Während der Fermentation müssen aerobe Verhältnisse sichergestellt werden; ein Sauerstoff-Partialdruckes von pO2 ≥ 20 % ist einzuhalten.

Die Fermentation ist nach 48 ± 2 Stunden beendet und wird durch Zusatz von 0,225 ± 0,025 % Benzalkoniumchlorid, z.B. 0,45 ± 0,05 % ®Dodigen 226 (einer 50 % igen Lösung einer Mischung von Alkyl-dimethyl-benzylammoniumchloriden in Wasser) beendet.

Anschließend beginnt die Aufarbeitung zur Isolierung von Hirudin aus der Kulturbrühe.

Das Endvolumen (Medium + Kondensat + Vorkultur + zudosierter Glucose-Lösung - Wasserverlust infolge Begasung) beträgt 2.300 ± 50 l.

### b) Batch-Prozeß

Der batch-Prozeß ist dem unter a) beschriebenen fed-batch-Prozeß äquivalent mit folgenden Ausnahmen:
- Das Medium enthält vor dem Beimpfen neben den komplexen Bestandteilen auch 4 % Glucose.
- Es erfolgt keine weitere Zudosierung von Glucose.
- Das Endvolumen beträgt nur ca. 1.800 ± 50 l.

### c) Vergleich der Ergebnisse

| Verfahren | Hirudin-Konzentration [%] | Hirudin-Menge [%] |
|---|---|---|
| batch Prozeß | 100 | 100 |
| fed-batch Prozeß | 228 | 291 |

## Patentansprüche

1. Verfahren zur biotechnologischen Fermentation von Hirudin, Hirudin-Derivaten oder Fusionsproteinen enthaltend Hirudin oder ein Hirudin-Derivat in Hefe unter Benutzung eines pα-ADH II Promotorsystems, **dadurch gekennzeichnet, daß** man
a) eine Hauptkultur mit einer Vorkultur eines Hefestammes, der das Hirudin, Hirudin-Derivat oder Fusionsprotein enthaltend Hirudin oder ein Hirudin-Derivat exprimieren kann, animpft;
b) unmittelbar nach dem Animpfen 0,7-1,4 g Glucose/l Kulturvolumen/h mit konstanter Rate kontinuierlich zuführt,
c) aerobe Kulturbedingungen sicherstellt;
d) die Fermentation nach ca. 2 Tagen beendet und schließlich
e) das Hirudin, Hirudin-Derivat oder Fusionsprotein enthaltend Hirudin oder ein Hirudin-Derivat aus dem Fermentationsansatz isoliert.

2. Verfahren nach Anspruch 1, wobei die Glucose unmittelbar nach dem Animpfen mit konstanter Rate von 0,9-1,3 oder mit 1,1 g Glucose/l Kulturvolumen/h kontinuierlich zugeführt wird.

3. Verfahren nach Anspruch 1, **dadurch gekennzeichnet, daß** die Medien der Vor- und Hauptkultur, bis auf einen 1%igen Glucosezusatz im Vorkulturstadium, äquivalent sind.

4. Verfahren nach einem der Ansprüche 1 oder 3, **dadurch gekennzeichnet, daß** der 1%ige Glucosezusatz im Vorkulturmedium zum Zeitpunkt des Beimpfens praktisch völlig verbraucht ist.

5. Verfahren nach einem der Ansprüche 1 bis 4, **dadurch gekennzeichnet, daß** die aeroben Kulturbedingungen durch einen Sauerstoffpartialdruck von mindestens 20% der Sättigung sichergestellt werden.

6. Verfahren nach einem der Ansprüche 1 bis 5, **dadurch gekennzeichnet, daß** die genannten Medien einen oder mehrere komplexe Bestandteile, wie z.B. Cornsteep, Sojamehl oder Hefeextrakt enthalten.

7. Verfahren nach einem der Ansprüche 1 bis 5, **dadurch gekennzeichnet, daß** der genannte Hefestamm ein Saccharomyces cerevisiae-Stamm, vorzugweise Y79, ist.

8. Verfahren nach einem der Ansprüche 1 bis 6, **dadurch gekennzeichnet, daß** der genannte Hefestamm ein Kluyveromyces lactis-Stamm ist.

## Claims

1. A process for the biotechnological fermentation of hirudin, hirudin derivatives or fusion proteins containing hirudin or a hirudin derivative in yeast using a pα-ADH II promoter system, which comprises
a) inoculating a main culture with a preliminary culture of a yeast strain which can express the hirudin, hirudin derivatives or fusion protein containing hirudin or a hirudin derivative;
b) immediately after the inoculation supplying 0.7-1.4 g of glucose/l of culture volume/h continuously at a constant rate;
c) ensuring aerobic culture conditions;
d) terminating the fermentation after approx. 2 days, and finally,
e) isolating the hirudin, hirudin derivatives or fusion protein containing hirudin or a hirudin derivative from the fermentation culture.

2. The process as claimed in claim 1, the glucose being supplied immediately after the inoculation continuously at a constant rate of 0.9 -1.3 or at 1.1 g of glucose/I of culture volume/h.

3. The process as claimed in claim 1, wherein the media of the preliminary culture and the main culture are equivalent apart from an addition of 1% glucose to the preliminary culture medium.

4. The process as claimed in one of claims 1 and 3, wherein the 1% glucose which has been added to the preliminary culture medium has been almost completely consumed at the time of the inoculation.

5. The process as claimed in one of claims 1 to 4, wherein the aerobic culture conditions are ensured by means of an oxygen partial pressure of at least 20% saturation.

6. The process as claimed in one of claims 1 to 5, wherein said media contain one or more complex constituents, for example cornsteep, soyabean meal or yeast extract.

7. The process as claimed in one of claims 1 to 5, wherein said yeast strain is a Saccharomyces cerevisiae strain, preferably Y79.

8. The process as claimed in one of claims 1 to 6, wherein said yeast strain is a Kluyveromyces lactis strain.

## Revendications

1. Procédé de fermentation biotechnologique de l'hirudine, de dérivés de l'hirudine ou de protéines de fusion renfermant de l'hirudine ou d'un dérivé de l'hirudine dans de la levure en utilisant un système promoteur pα-ADH II, **caractérisé en ce que**
a) une culture principale est inoculée avec une préculture d'une souche de levure qui peut exprimer l'hirudine, un dérivé de l'hirudine ou une protéine de fusion renfermant de l'hirudine ou un dérivé de l'hirudine ;
b) de 0,7 à 1,4 g de glucose/l de volume de culture/h est introduit en continu à une vitesse constante, immédiatement après l'inoculation ;
c) des conditions de culture aérobies sont assurées ;
d) la fermentation est terminée après environ 2 jours, et enfin
e) l'hirudine, le dérivé de l'hirudine ou la protéine de fusion renfermant de l'hirudine ou un dérivé de l'hirudine est isolé du mélange de fermentation.

2. Procédé selon la revendication 1, dans lequel le glucose est introduit en continu, immédiatement après l'inoculation, à une vitesse constante de 0,9 à 1,3 ou à 1,1 g de glucose/l de volume de culture/h.

3. Procédé selon la revendication 1, **caractérisé en ce que** les milieux de la préculture et de la culture principale sont équivalents, mis à part l'addition de 1 % de glucose au stade de la préculture.

4. Procédé selon l'une quelconque des revendications 1 ou 3, **caractérisé en ce que** l'addition de 1 % de glucose dans le milieu de préculture est presque entièrement consommée au moment de l'inoculation.

5. Procédé selon l'une quelconque des revendications 1 à 4, **caractérisé en ce que** les conditions de culture aérobies sont assurées par une pression partielle d'oxygène d'au moins 20 % de la saturation.

6. Procédé selon l'une quelconque des revendications 1 à 5, **caractérisé en ce que** lesdits milieux contiennent un ou plusieurs constituants complexes tels que, par exemple, l'extrait soluble de maïs, la farine de soja ou l'extrait de levure.

7. Procédé selon l'une quelconque des revendications 1 à 5, **caractérisé en ce que** ladite souche de levure est une souche de Saccharomyces cerevisiae, de préférence Y79.

8. Procédé selon l'une quelconque des revendications 1 à 6, **caractérisé en ce que** ladite souche de levure est une souche de Kluyveromyces lactis.
